(19)

Europäisches
Patentamt
European
Patent Office
Office européen
des brevets

(11) **EP 2 826 482 A1**

(12) **EUROPEAN PATENT APPLICATION**
published in accordance with Art. 153(4) EPC

(43) Date of publication:
**21.01.2015 Bulletin 2015/04**

(21) Application number: **13760458.3**

(22) Date of filing: **15.03.2013**

(51) Int Cl.:
*A61K 31/53* (2006.01)     *A61K 31/282* (2006.01)
*A61K 31/337* (2006.01)     *A61K 31/4412* (2006.01)
*A61K 31/513* (2006.01)     *A61P 35/00* (2006.01)
*A61P 43/00* (2006.01)

(86) International application number:
**PCT/JP2013/057403**

(87) International publication number:
**WO 2013/137433 (19.09.2013 Gazette 2013/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **16.03.2012 JP 2012059987**

(71) Applicant: **Taiho Pharmaceutical Co., Ltd.**
**Chiyoda-ku**
**Tokyo 101-8444 (JP)**

(72) Inventor: **UCHIDA, Junji**
**Tokushima-shi**
**Tokushima 771-0194 (JP)**

(74) Representative: **Hoffmann Eitle**
**Patent- und Rechtsanwälte PartmbB**
**Arabellastraße 30**
**81925 München (DE)**

(54) **NOVEL ANTITUMOR AGENT COMPRISING COMBINATION OF THREE AGENTS**

(57)     A primary object of the present invention is to provide a novel combination therapy that exhibits a notable antitumor effect.

As means for achieving the object, an antitumor agent comprising a combination of oxaliplatin, paclitaxel, and a combination drug containing tegafur, gimeracil, and oteracil potassium is provided.

EP 2 826 482 A1

**Description**

Technical Field

Cross-Reference to Related Applications

The present application claims priority to the specification of Japan Patent Application No. 2012-059987 (the entire disclosure of which is incorporated in the present specification by reference) filed on March 16, 2012.

The present invention relates to a novel antitumor agent comprising oxaliplatin (hereafter may be referred to as "l-OHP"), paclitaxel, and a combination drug containing tegafur, gimeracil, and oteracil potassium.

Background Art

Antitumor agents, such as 5-fluorouracil (hereafter "5-FU"), cisplatin, irinotecan, paclitaxel, docetaxel, a combination drug of tegafur and uracil (product name: UFT®), a combination drug of tegafur, gimeracil, and oteracil potassium (product name: TS-1®; hereafter, a preparation containing tegafur, gimeracil, and oteracil potassium at a molar ratio of 1:0.4:1 is referred to as TS-1), used singly or in combination, have been clinically applied in chemotherapy for advanced or recurrent gastric cancer in Japan and abroad. A combination chemotherapy of 5-FU and cisplatin is used as a standard therapy in Europe and the U.S.; however, its life-prolongation effect is still not satisfactory. Therefore, a combination chemotherapy of TS-1 and cisplatin is expected to be a combination chemotherapy that exhibits a superior life-prolongation effect (Non-patent Literature 1).

Further, various combination therapies have been developed to enhance the antitumor effect of TS-1, and it has been reported that a combination therapy of TS-1 with l-OHP exhibits a high therapeutic effect (Patent Literature 1 and Non-patent Literature 2). The combination therapy of TS-1 and l-OHP is a useful therapy that attains a high therapeutic effect on, for example, advanced and recurrent colorectal cancer. However, a therapy with a higher antitumor effect and lower side effects is desired to also contribute to the long-term survival of patients with gastric cancer.

Meanwhile, paclitaxel is known as an antitumor agent that belongs to the taxanes, which inhibit tumor cell division by binding to and stabilizing microtubules to thereby inhibit the depolymerization of the microtubules. Additionally, it has been reported that the combined use of TS-1 and paclitaxel enhances antitumor effects (Non-patent Literature 3). However, this also still does not exhibit a sufficiently satisfactory antitumor effect. There is thus a demand for a therapy that contributes to long-term survival, and that has lower side effects.

Citation List

Patent Literature

PTL 1: WO05/120480

Non-patent Literature

NPL 1: Cancer 2007; 109: 33-40.
NPL 2: J. Clin. Oncol. 2009; 27 (15s): 4553.
NPL 3: Oncology 2008; 74 (1-2): 37-41.

Summary of Invention

Technical Problem

An object of the present invention is to provide a novel antitumor agent and antitumor therapy that exhibit a notable antitumor effect, in particular, an effect of tumor growth delay.

Solution to Problem

In view of the current state of the art described above, the present inventor conducted extensive research on a novel combination therapy in which a combination therapy of TS-1 and 1-OHP (hereafter, "TS-1/l-OHP combination therapy") is used in combination with another antitumor agent, in order to develop a tumor therapy that further contributes to the prolongation of the survival of patients. As a result, the inventor found that when the TS-1/l-OHP combination therapy is used in combination with paclitaxel, the antitumor effect of the TS-1/l-OHP combination therapy is notably enhanced, whereas the development of side effects is not increased. The present invention has been accomplished based on this finding. In general, when one antitumor agent is used in combination with another antitumor agent, the antitumor effect is enhanced, but the side effects are also increased. Therefore, it is surprising that both a notably high antitumor effect, in particular, an effect of tumor growth delay, and a reduced risk of developing side effects can be achieved as in the present invention.

Specifically, the present invention encompasses the following inventions 1) to 33).

1) An antitumor agent comprising a combination of oxaliplatin, paclitaxel, and a combination drug containing tegafur, gimeracil, and oteracil potassium.

2) The antitumor agent according to 1), wherein the combination drug contains tegafur, gimeracil, and oteracil potassium at a molar ratio of tegafur:gimeracil:oteracil potassium = 1:0.4:1.

3) The antitumor agent according to any one of 1) and 2), which is in a pharmaceutical form comprising the combination drug containing tegafur, gimeracil, and oteracil potassium, a preparation containing oxaliplatin, and a preparation containing paclitaxel.

4) The antitumor agent according to 3), wherein the combination drug containing tegafur, gimeracil, and oteracil potassium is administered by oral administration, and the preparation containing oxaliplatin and the preparation containing paclitaxel are individually administered by an administration route selected from the group consisting of intravenously, intramuscularly, and subcutaneously.

5) The antitumor agent according to any one of 3) and 4), wherein the combination drug containing tegafur, gimeracil, and oteracil potassium, the preparation containing oxaliplatin, and the preparation containing paclitaxel are administered simultaneously or at one or more intervals.

6) The antitumor agent according to any one of 1) to 5), wherein oxaliplatin is contained at a daily dose of 80 to 150 mg/m$^2$, paclitaxel is contained at a daily dose of 50 to 500 mg/m$^2$, and the combination drug containing tegafur, gimeracil, and oteracil potassium is contained at a daily dose such that tegafur is contained in an amount of 60 to 120 mg/m$^2$.

7) The antitumor agent according to any one of 3) to 6), wherein the pharmaceutical form of paclitaxel is a nanoparticle preparation comprising paclitaxel encapsulated in albumin.

8) A kit comprising a combination drug containing tegafur, gimeracil, and oteracil potassium, a preparation containing oxaliplatin, and a preparation containing paclitaxel.

9) The kit according to 8), wherein the combination drug contains tegafur, gimeracil, and oteracil potassium at a molar ratio of tegafur:gimeracil:oteracil potassium = 1:0.4:1.

10) The kit according to any one of 8) and 9), wherein the combination drug containing tegafur, gimeracil, and oteracil potassium is administered by oral administration, and the preparation containing oxaliplatin and the preparation containing paclitaxel are individually administered by an administration route selected from the group consisting of intravenously, intramuscularly, and subcutaneously.

11) The kit according to any one of 8) to 10), wherein the combination drug containing tegafur, gimeracil, and oteracil potassium, the preparation containing oxaliplatin, and the preparation containing paclitaxel are administered simultaneously or at one or more intervals.

12) The kit according to any one of 8) to 11), wherein oxaliplatin is contained at a daily dose of 80 to 150 mg/m$^2$, paclitaxel is contained at a daily dose of 50 to 500 mg/m$^2$, and the combination drug containing tegafur, gimeracil, and oteracil potassium is contained at a daily dose such that tegafur is contained in an amount of 60 to 120 mg/m$^2$.

13) The kit according to any one of 8) to 12), wherein the pharmaceutical form of paclitaxel is a nanoparticle preparation comprising paclitaxel encapsulated in albumin.

14) A method for treating a tumor, comprising administering oxaliplatin, paclitaxel, and a combination drug containing tegafur, gimeracil, and oteracil potassium, in combination.

15) The method according to 14), wherein tegafur, gimeracil, and oteracil potassium of the combination drug are administered at a molar ratio of tegafur:gimeracil:oteracil potassium = 1:0.4:1.

16) The method according to any one of 14) and 15), wherein a pharmaceutical form comprising the combination drug containing tegafur, gimeracil, and oteracil potassium, a preparation containing oxaliplatin, and a preparation containing paclitaxel is used.

17) The method according to 16), wherein the combination drug containing tegafur, gimeracil, and oteracil potassium is administered by oral administration, and the preparation containing oxaliplatin and the preparation containing paclitaxel are individually administered by an administration route selected from the group consisting of intravenously, intramuscularly, and subcutaneously.

**EP 2 826 482 A1**

18) The method according to any one of 16) and 17), wherein the combination drug containing tegafur, gimeracil, and oteracil potassium, the preparation containing oxaliplatin, and the preparation containing paclitaxel are administered simultaneously or at one or more intervals.

19) The method according to any one of 14) to 18), wherein oxaliplatin is used at a daily dose of 80 to 150 mg/m$^2$, paclitaxel is used at a daily dose of 50 to 500 mg/m$^2$, and the combination drug containing tegafur, gimeracil, and oteracil potassium is used at a daily dose such that tegafur is used in an amount of 60 to 120 mg/m$^2$.

20) The method according to any one of 16) to 19), wherein the pharmaceutical form of paclitaxel is a nanoparticle preparation comprising paclitaxel encapsulated in albumin.

21) Use of tegafur, gimeracil, oteracil potassium, oxaliplatin, and paclitaxel for tumour treatment that uses oxaliplatin, paclitaxel, and a combination drug containing tegafur, gimeracil, and oteracil potassium, in combination.

22) A combination of pharmaceutical compositions for tumor treatment, the combination comprising an antitumor composition containing tegafur, gimeracil, and oteracil potassium, an antitumor composition containing oxaliplatin, and an antitumor composition containing paclitaxel.

23) A combination drug containing tegafur, gimeracil, and oteracil potassium for use in tumor treatment in combination with oxaliplatin and paclitaxel.

24) A combination drug containing tegafur, gimeracil, and oteracil potassium for treating a patient with a tumor by administering the combination drug, oxaliplatin, and paclitaxel simultaneously or at one or more intervals.

25) A kit comprising a combination drug containing tegafur, gimeracil, and oteracil potassium, the kit further comprising a package insert, label, or instructions each stating that the combination drug, oxaliplatin, and paclitaxel are administered in combination for treating a patient with a tumor.

26) Paclitaxel for use in tumor treatment in combination with oxaliplatin and a combination drug containing tegafur, gimeracil, and oteracil potassium.

27) Paclitaxel for treating a patient with a tumor by administering paclitaxel, oxaliplatin and a combination drug containing tegafur, gimeracil, and oteracil potassium simultaneously or at one or more intervals.

28) Paclitaxel according to any one of 26) and 27), wherein the pharmaceutical form is a nanoparticle preparation comprising paclitaxel encapsulated in albumin.

29) A kit comprising paclitaxel, the kit further comprising a package insert, label, or instructions each stating that paclitaxel, oxaliplatin, and a combination drug containing tegafur, gimeracil, and oteracil potassium are administered in combination for treating a patient with a tumor.

30) The kit according to 29), wherein the pharmaceutical form of paclitaxel is a nanoparticle preparation comprising paclitaxel encapsulated in albumin.

31) Use of oxaliplatin, paclitaxel, and a combination drug containing tegafur, gimeracil, and oteracil potassium for the production of a medicament for treating a tumor.

32) Use of a combination drug containing tegafur, gimeracil, and oteracil potassium for the production of a medicament for treating a tumor in combination with oxaliplatin and paclitaxel.

33) Use of paclitaxel for the production of a medicament for treating a tumor in combination with oxaliplatin and a combination drug containing tegafur, gimeracil, and oteracil potassium.

Further, the present invention relates to the following inventions.

The antitumor agent according to 1), which is in a pharmaceutical form comprising a plurality of preparations each containing one or more active ingredients selected from the group consisting of oxaliplatin, paclitaxel, and the combination drug containing tegafur, gimeracil, and oteracil potassium; or in a pharmaceutical form comprising a single preparation containing all of the active ingredients.

The antitumor agent according to 1), which is in a pharmaceutical form comprising a preparation containing (1) three ingredients, i.e., tegafur, gimeracil, and oteracil potassium as active ingredients and a preparation containing (2) oxaliplatin and paclitaxel as active ingredients.

The antitumor agent according to 1), wherein the proportions of the active ingredients are such that, per mole of tegafur, gimeracil is used in an amount of 0.1 to 5 moles, oteracil potassium is used in an amount of 0.1 to 5 moles, oxaliplatin is used in an amount of 0.1 to 5 moles, and paclitaxel is used in an amount of 0.01 to 100 moles.

The antitumor agent according to 1), wherein the molar ratio of the active ingredients is tegafur:gimeracil:oteracil potassium:oxaliplatin:paclitaxel = 1:0.4:1:0.4 to 2:0.3 to 10.

The antitumor agent according to 1), wherein the antitumor agent is in the form of a kit comprising (1) the combination drug containing tegafur, gimeracil, and oteracil potassium, (2) a preparation containing oxaliplatin, and (3) a preparation containing paclitaxel.

A kit comprising a combination of pharmaceutical compositions for treating a tumor in a mammal, the combination comprising (a) an antitumor composition containing tegafur in a therapeutically effective amount, gimeracil in an amount effective for enhancing antitumor effect, and oteracil potassium in an amount effective for inhibiting side effect, (b) an antitumor composition containing oxaliplatin in a therapeutically effective amount, and (c) an antitumor composition

4

containing paclitaxel in a therapeutically effective amount.

A method for treating a tumor, comprising administering, to a mammal, in particular, a human, (1) a therapeutically effective amount of a combination drug containing tegafur, gimeracil, and oteracil potassium, (2) a therapeutically effective amount of oxaliplatin and (3) a therapeutically effective amount of paclitaxel, in combination.

Advantageous Effects of Invention

The antitumor agent of the present invention, which comprises a combination of l-OHP, paclitaxel, and a combination drug containing tegafur, gimeracil, and oteracil potassium, exhibits a notably high antitumor effect, such as reduction in tumor volume or delay of tumor growth, as compared with the hitherto known combined use of antitumor agents. Thus, the use of the antitumor agent of the present invention can provide a longer survival time for patients.

Description of Embodiments

As shown in the below-described Examples, the antitumor agent of the present invention, which comprises l-OHP, paclitaxel, and a combination drug containing tegafur, gimeracil, and oteracil potassium, in combination, can achieve a notably high antitumor effect while suppressing side effects, as compared with cases in which they are used singly or in which two of them are used in combination. In the present invention, the antitumor effect is evaluated as a tumor-shrinking effect, an effect of delaying tumor growth, or the like.

In the present invention, examples of the combination drug containing tegafur, gimeracil, and oteracil potassium include a preparation prepared by mixing tegafur, gimeracil, and oteracil potassium in any proportion, and suitably adding the below-described pharmaceutically acceptable carriers thereto.

Here, "tegafur" is a known compound denoted as 5-fluoro-1-(2-tetrahydrofuryl)-2,4-(1H,3H)-pyrimidinedione. Tegafur is a drug that is activated *in vivo* to release 5-FU, which is a substance responsible for antitumor activity. Tegafur can be produced according to known methods, for example, the method disclosed in JPS49-010510B.

"Gimeracil" is a known compound denoted as 2,4-dihydroxy-5-chloropyridine. Although gimeracil itself does not exhibit any antitumor activity, it can inhibit metabolic inactivation of 5-FU *in vivo,* thereby enhancing the antitumor effect.

"Oteracil potassium" is a known compound denoted as monopotassium 1,2,3,4-tetrahydro-2,4-dioxo-1,3,5-triazine-6-carboxylate. Although oteracil potassium itself does not exhibit any antitumor activity, it is chiefly distributed in the gastrointestinal tract, where it inhibits the activation of 5-FU, thereby preventing gastrointestinal tract disorders.

The proportions of tegafur, gimeracil, and oteracil potassium are not particularly limited, as long as the purpose of each ingredient is achieved. For example, the proportions of tegafur, gimeracil and oteracil potassium may be within the same range as those in the known combination drug disclosed in JP2614164B. The proportions are usually such that, per mole of tegafur, gimeracil is used in an amount of about 0.1 to about 5 moles and preferably about 0.2 to about 1.5 moles, and oteracil potassium is used in an amount of about 0.1 to about 5 moles and preferably about 0.2 to about 2 moles. The proportions of the active ingredients are particularly preferably such that the molar ratio of tegafur:gimeracil:oteracil potassium is 1:0.4:1. Examples of the combination drug comprising the ingredients at the aforementioned molar ratio include a commercially available "combination drug of tegafur, gimeracil, and oteracil potassium," a generic name (product name: "TS-1," Taiho Pharmaceutical Co. Ltd.).

The "oxaliplatin" (l-OHP) of the present invention is a known compound denoted as cis-oxalate(1R,2R-diaminocyclohexane) platinum (II) . l-OHP binds to the DNA of tumor cells to induce DNA dysfunction and DNA strand breakage, thereby exerting the action of annihilating the tumor cells. l-OHP can be produced according to known methods, for example, the method disclosed in JPS60-041077B.

The proportion of l-OHP in the antitumor agent of the present invention is not particularly limited as long as the antitumor effect is enhanced. For example, the proportion of 1-OHP is usually such that, per mole of tegafur, l-OHP is used in an amount of about 0.1 to about 5.0 moles, preferably about 0.2 to about 3.0 moles, and more preferably about 0.4 to about 2.0 moles as a daily dose.

The "paclitaxel" of the present invention is a known compound that exerts an antitumor effect by inhibiting the depolymerization of the microtubles of the cytoskeleton to arrest the cell cycle at G2/M phase, thereby inhibiting cell division. Paclitaxel is known to exhibit an antitumor effect on ovarian cancer, non-small-cell lung cancer, breast cancer, gastric cancer, endometrial cancer, and the like.

The paclitaxel of the present invention encompasses derivatives and drug delivery system (DDS) preparations of paclitaxel intended to alleviate the side effects of paclitaxel. Examples thereof include an albumin-bound paclitaxel injectable suspension (product name "Abraxane"), which is a nanoparticle preparation comprising paclitaxel encapsulated in albumin; a polymeric micelle in which paclitaxel is encapsulated in a block copolymer of polyethylene glycol and polyaspartic acid (NK105); a prodrug in which paclitaxel is linked to docosahexaenoic acid (DHA), a fatty acid (Taxoprexin); a prodrug in which paclitaxel is linked to polyglutamic acid (product name "Opaxio"); a prodrug in which paclitaxel is linked to a monoclonal antibody that targets tumor cells; and the like.

A nanoparticle preparation comprising paclitaxel encapsulated in albumin, which is a preferred pharmaceutical form of paclitaxel of the present invention, can be produced according to, for example, the method disclosed in JP2003-300878A. As the nanoparticle preparation comprising paclitaxel encapsulated in albumin, a commercially available product such as an albumin-bound paclitaxel injectable suspension sold under the product name "Abraxane" is also usable.

The proportion of paclitaxel in the antitumor agent of the present invention is not particularly limited as long as the antitumor effect is enhanced. For example, the proportion of paclitaxel is usually such that, per mole of tegafur, paclitaxel is used in an amount of about 0.01 to about 100 moles, preferably about 0.1 to about 30 moles, and more preferably about 0.3 to about 10 moles as a daily dose.

The antitumor agent of the present invention may be formulated into a single dosage form (single-formulation type) as a combination drug (preparation containing a plurality of active ingredients) that contains l-OHP, paclitaxel, and the combination drug containing tegafur, gimeracil, and oteracil potassium at the above-described proportions; or may be formulated into a plurality of dosage forms (multiple-formulation type) by formulating the above-described active ingredients into single active ingredient preparations (preparations containing a single active ingredient) or combination drugs, to be used simultaneously or at one or more separate intervals. Of these, preferable is a multiple-formulation type in which a preparation containing l-OHP and a preparation containing paclitaxel are individually formulated as single active ingredient preparations and used in combination with a separately prepared combination drug containing tegafur, gimeracil, and oteracil potassium.

Tumors that can be treated with the antitumor agent of the present invention are not particularly limited. Examples thereof include colorectal cancer, liver cancer, kidney cancer, head and neck cancer, esophageal cancer, gastric cancer, biliary tract cancer, gallbladder and bile duct cancer, pancreatic cancer, lung cancer, breast cancer, ovarian cancer, cervical cancer, endometrial cancer, bladder cancer, prostatic cancer, testicular tumor, bone and soft tissue sarcomas, leukemia, malignant lymphoma, multiple myeloma, skin cancer, brain tumor, and the like. Of these, colorectal cancer, gastric cancer, head and neck cancer, lung cancer, breast cancer, pancreatic cancer, biliary tract cancer, and liver cancer are preferable. Colorectal cancer, gastric cancer, lung cancer, and breast cancer are particularly preferred.

The dosage forms of the preparations are not particularly limited, and can be suitably selected depending on the purpose of the treatment. Specific examples thereof include oral preparations (such as tablets, coated tablets, powders, granules, capsules, and fluids), injections, suppositories, patches, and ointments. When the antitumor agent of present invention is formulated into a plurality of dosage forms, the preparations may be presented in different dosage forms or in the same dosage form. For example, the combination drug containing tegafur, gimeracil, and oteracil potassium is preferably an oral preparation, and the preparation containing l-OHP and the preparation containing paclitaxel are preferably injections.

Usable unit dosage forms of the antitumor agent containing three ingredients, i.e., tegafur, gimeracil, and oteracil potassium, as active ingredients, for the treatment of malignant tumors in mammals including humans are not particularly limited, and can be suitably selected according to the purpose of the treatment. Specific examples thereof include parenteral forms such as injections, suppositories, ophthalmic solutions, ointments, aerosols, and the like; and oral forms such as tablets, coated tablets, powders, granules, capsules, fluids, pills, suspensions, emulsions, and the like. Of these, oral forms are preferable. The antitumor agent can be produced in such dosage forms according to methods commonly known in this technical field.

Usable unit dosage forms of the preparation containing l-OHP as an active ingredient for the treatment of malignant tumors in mammals including humans are not particularly limited, and can be suitably selected depending on the purpose of the treatment. Specific examples thereof include parenteral forms such as injections, suppositories, ophthalmic solutions, ointments, aerosols, and the like; and oral forms such as tablets, coated tablets, powders, granules, capsules, fluids, pills, suspensions, emulsions, and the like. Of these, injections are preferable. It is particularly preferred that the preparation containing l-OHP be administered intravenously, intramuscularly, or subcutaneously. The preparation can be produced in such dosage forms according to methods commonly known in this technical field.

Usable unit dosage forms of the preparation containing paclitaxel as an active ingredient for the treatment of malignant tumors in mammals including humans are not particularly limited, and can be suitably selected depending on the purpose of the treatment. Specific examples thereof include parenteral forms such as injections, suppositories, ophthalmic solutions, ointments, aerosols, and the like; and oral forms such as tablets, coated tablets, powders, granules, capsules, fluids, pills, suspensions, emulsions, and the like. Of these, injections are preferable. It is particularly preferred that the preparation containing paclitaxel be administered intravenously, intramuscularly, or subcutaneously. The preparation can be produced in such dosage forms according to methods commonly known in this technical field.

Regarding the antitumor agent of the present invention, as long as l-OHP, paclitaxel, and the combination drug containing tegafur, gimeracil, and oteracil potassium are administered in combination, each of the preparations described above may be individually produced, packed, and distributed; or all or a part of the preparations may be produced, packed, and distributed as a single package (kit formulation) suitable for administering in combination.

The preparations containing the active ingredients of the present invention can be produced using pharmaceutically acceptable carriers by commonly known methods. Examples of carriers include those that are widely used in common drugs, such as excipients, binders, disintegrators, lubricants, diluents, solubilizing agents, suspending agents, tonicity adjusting agents, pH adjusters, buffers, stabilizers, colorants, sweetening agents, flavoring agents, and the like.

Examples of excipients include lactose, saccharose, sodium chloride, glucose, maltose, mannitol, erythritol, xylitol, maltitol, inositol, dextran, sorbitol, albumin, urea, starch, calcium carbonate, kaolin, crystalline cellulose, silicic acid, methylcellulose, glycerol, sodium alginate, gum arabic, and mixtures thereof. Examples of lubricants include purified talc, stearic acid salts, borax, polyethylene glycol, and mixtures thereof. Examples of binders include simple syrups, glucose solutions, starch solutions, gelatin solutions, polyvinyl alcohol, polyvinyl ether, polyvinylpyrrolidone, carboxymethylcellulose, shellac, methylcellulose, ethylcellulose, water, ethanol, potassium phosphate, and mixtures thereof. Examples of disintegrators include dried starch, sodium alginate, powdered agar, powdered laminaran, sodium hydrogencarbonate, calcium carbonate, polyoxyethylene sorbitan fatty acid esters, sodium lauryl sulfate, stearic acid monoglycerides, starch, lactose, and mixtures thereof. Examples of diluents include water, ethyl alcohol, macrogol, propylene glycol, ethoxylated isostearyl alcohol, polyoxylated isostearyl alcohol, polyoxyethylene sorbitan fatty acid esters, and mixtures thereof. Examples of stabilizers include sodium pyrosulfite, ethylene diamine tetraacetic acid, thioglycolic acid, thiolactic acid, and mixtures thereof. Examples of tonicity adjusting agents include sodium chloride, boric acid, glucose, glycerol, and mixtures thereof. Examples of pH adjusters and buffers include sodium citrate, citric acid, sodium acetate, sodium phosphate, and mixtures thereof. Examples of soothing agents include procaine hydrochloride, lidocaine hydrochloride and mixtures thereof.

The dose of each active ingredient in the antitumor agent of the present invention is not particularly limited as long as the purpose of each active ingredient is achieved, and can be suitably determined according to age of a patient, cancer type, stage of disease, presence or absence of metastasis, medical history, presence or absence of other antitumor agents, and other factors. Specific examples of the dose of each active ingredient include the following: the amount of tegafur is 20 to 500 mg/m$^2$ (per body surface area)/day and preferably 60 to 120 mg/m$^2$/day; the amount of gimeracil is 5.8 to 145 mg/m$^2$/day and preferably 17.4 to 34.8 mg/m$^2$/day; the amount of oteracil potassium is 19.6 to 490 mg/m$^2$/day and preferably 58.8 to 117.6 mg/m$^2$/day; the amount of l-OHP is 40 to 300 mg/m$^2$/day and preferably 80 to 150 mg/m$^2$/day; and the amount of paclitaxel is 10 to 1000 mg/m$^2$/day and preferably 50 to 500 mg/m$^2$/day.

Accordingly, it is preferable that the antitumor agent of the present invention contain l-OHP, paclitaxel, and the combination drug containing tegafur, gimeracil, and oteracil potassium such that the daily doses are within the aforementioned ranges.

More specifically, it is preferable that the combination drug containing tegafur, gimeracil, and oteracil potassium be contained at a daily dose such that tegafur is contained in an amount of 20 to 500 mg/m$^2$ and preferably 60 to 120 mg/m$^2$, that l-OHP be contained at a daily dose of 40 to 300 mg/m$^2$ and preferably 80 to 150 mg/m$^2$, and that paclitaxel be contained at a daily dose of 10 to 1000 mg/m$^2$ and preferably 50 to 500 mg/m$^2$.

The administration schedule (order of administration and administration intervals) of the active ingredients in the present invention is not particularly limited as long as a synergistic effect is obtained.

In addition, when the antitumor agent of the present invention is provided as a kit, individual preparations may be administered simultaneously or at one or more intervals.

In the present invention, the combination drug containing tegafur, gimeracil, and oteracil potassium, the preparation containing l-OHP, and the preparation containing paclitaxel may be packaged in a kit comprising a combination of pharmaceutical compositions for treating tumors in mammals, the combination comprising (a) an antitumor composition containing tegafur in a therapeutically effective amount, gimeracil in an amount effective for enhancing antitumor effect, and oteracil potassium in an amount effective for inhibiting side effect, (b) a composition containing l-OHP in a therapeutically effective amount, and (c) a composition containing paclitaxel in a therapeutically effective amount. The compositions contained in the kit may be in any known pharmaceutical form. The compositions are usually placed in commonly used containers according to their pharmaceutical form.

In the present invention, the kit comprising a combination of pharmaceutical compositions for treating tumors means a kit comprising the pharmaceutical compositions in any combination, or a kit comprising all of the active ingredients in a single form.

The kit may be a kit for treating tumors in mammals, comprising, for example, at least the above-mentioned three compositions (a) to (c), and at least two containers for these compositions, wherein the compositions (a) and (c) are contained in different containers. The compositions (a) to (c) are preferably in a pharmaceutical form prepared in combination with pharmaceutically acceptable carriers. Provided that the kit comprises the compositions (a) and (c) in different containers, the composition (b) may be accommodated in a container different from the other two compositions, or may be accommodated in the same container together with the composition (a) or (c) as a mixture.

Further, the kit may comprise a package insert, label, instructions, or the like. The package insert, label, instructions, or the like may state, for example, that at least, the above-mentioned three compositions (a) to (c) are administered in combination.

Examples

Examples are given below to illustrate the present invention in more detail; however, the present invention is not limited to these Examples.

Example 1

Effect of combined use with paclitaxel on the antitumor effect of TS-1+l-OHP with respect to human gastric cancer cell line SC-2

[Formulation of pharmaceutical preparations]

A TS-1 preparation was prepared by suspending a combination drug of tegafur, gimeracil, and oteracil potassium (tegafur:gimeracil:oteracil potassium (molar ratio) = 1:0.4:1, TS-1) in a 0.5% hydroxypropylmethylcellulose (HPMC) aqueous solution.
A l-OHP preparation was prepared by dissolving l-OHP in a 5% glucose solution for injection (Otsuka Glucose, produced by Otsuka Pharmaceutical Factory Inc.).
A paclitaxel preparation (hereafter may be referred to as "TXL") was prepared by dissolving paclitaxel in a mixture of equal volumes of Cremophor® EL and ethanol, and diluting the resulting mixture with physiological saline immediately before administration. According to the package insert of a commercially available pharmaceutical preparation of docetaxel, a dissolution solution included therein was added to an undiluted solution of docetaxel and the resulting mixture was diluted with physiological saline immediately before administration, thereby preparing a docetaxel preparation (hereafter may be referred to as "TXT").

[Administration of preparations]

2-mm-cubic fragments of human gastric cancer SC-2 cell line were subcutaneously implanted in the backs of male nude BALB/c-nu/nu mice. When the mean value of tumor volumes (= 0.5 × major axis (mm) × minor axis (mm) × minor axis (mm)) reached about 160 mm$^3$, the mice were divided into groups (seven mice per group, day 0).
The TS-1 preparation was orally administered once a day at a dose level (indicated as the amount of tegafur) shown in Table 1 below for 14 consecutive days from the day after grouping (day 1). The l-OHP preparation (5 mg/kg), the paclitaxel preparation (10 mg/kg and 20 mg/kg), and the docetaxel preparation (10 mg/kg) were individually administered into the tail vein on the day after grouping (day 1) and on day 8.
The ratio of tumor volume on day 15 to tumor volume upon grouping (day 0) (tumor volume on day 15/tumor volume on day 0) was regarded as the relative tumor volume (RTV). Tumor growth inhibition (%) was determined using the mean relative tumor volume of each drug-administered group and the mean relative tumor volume of the tumor-bearing control group (not treated with drugs) according to the following formula 1.

Formula 1

(1-(relative tumor volume of drug-administered group)/(relative tumor volume of tumor-bearing control group)) × 100 (%)

The ratio of the difference in body weight between the mice on day 15 and the mice upon grouping (day 0) to the body weight of the mice on day 0 (body weight change; BWC, %) was determined as an index of systemic toxicity according to formula 2 below.

Formula 2

((Body weight of mice on day 15 – body weight of mice on day 0)/ body weight of mice on day 0) × 100(%)

Table 1 shows the obtained results.

Table 1

| Drug | TS-1 (Tegafur) Dose (mg/kg) | I-OHP Dose (mg/kg) | Paclitaxel or Docetaxel Dose (mg/kg) | Relative Tumor Volume (Mean) | Tumor Growth Inhibition (%) | Body Weight Change (%) |
|---|---|---|---|---|---|---|
| None | - | - | - | 15.88 | - | 4.81 |
| Tegafur + Gimeracil+ Oteracil Potassium+ Oxaliplatin (TS-1+I-OHP) | 5.0 | 5.0 | - | 8.12** | 48.8 | -5.43 |
| Tegafur + Gimeracil + Oteracil Potassium+ Oxaliplatin+ Paclitaxel (TS-1+I-OHP+TXL) | 5.0 | 5.0 | 10.0 | 4.71** | 70.3 | -1.89 |
| Tegafur + Gimeracil + Oteracil Potassium+ Oxaliplatin+ Paclitaxel (TS-1+I-OHP+TXL) | 5.0 | 5.0 | 20.0 | 2.66** | 83.2 | 2.65 |
| Tegafur + Gimeracil+ Oteracil Potassium+ Oxaliplatin+ Docetaxel (TS-1+I-OHP+TXT) | 5.0 | 5.0 | 10.0 | 2.35** | 85.2 | -14.3** |
| The symbol "**" indicates significant difference from the control (not treated with drugs) ($p<0.01$) | | | | | | |

Antitumor effect of TS-1/I-OHP/paclitaxel combination in human gastric cancer SC-2 xenograft model

The results show that when paclitaxel was administered in combination with the TS-1/I-OHP combination therapy, a statistically significantly high antitumor effect was obtained, and toxicity (in particular, an increase in toxicity) that was evaluated by body weight loss was not observed. On the other hand, when docetaxel was administered in combination, a similar antitumor effect was obtained, but toxicity (in particular, an increase in toxicity) was observed. These results suggest that the combined use of the TS-1/I-OHP combination therapy and paclitaxel achieves an excellent balance between antitumor effect and toxicity, and is a very useful therapy.

Example 2

Effect of combined use with paclitaxel on the antitumor effect of TS-1+I-OHP with respect to human gastric cancer cell line SC-2

[Formulation of pharmaceutical preparations]

A TS-1 preparation was prepared by suspending a combination drug of tegafur, gimeracil, and oteracil potassium (tegafur:gimeracil:oteracil potassium (molar ratio) = 1:0.4:1, TS-1) in a 0.5% hydroxypropylmethylcellulose (HPMC) aqueous solution.
A I-OHP preparation was prepared by dissolving I-OHP in a 5% glucose solution for injection (Otsuka Glucose, produced by Otsuka Pharmaceutical Factory Inc.).
A lyophilized powder of human serum albumin-bound nanoparticle paclitaxel was suspended in physiological saline, and the resulting mixture was used as a human serum albumin-bound nanoparticle paclitaxel preparation (hereafter, "APX preparation").

[Administration of preparations]

2-mm-cubic fragments of human gastric cancer SC-2 cell line were subcutaneously implanted in the backs of male nude BALB/c-nu/nu mice. When the mean value of tumor volumes (= 0.5 $\times$ major axis (mm) $\times$ minor axis (mm) $\times$ minor axis (mm)) reached about 120 mm$^3$, the mice were divided into groups (six mice per group, day 0).
The TS-1 preparation was orally administered once a day at a dose level (indicated as the amount of tegafur) shown in Table 2 below for 14 consecutive days from the day after grouping (day 1). The I-OHP preparation was administered in an amount of 5 mg/kg into the tail vein on the day after grouping (day 1) and on day 8. The APX preparation was administered in an amount of 20 mg/kg or 40 mg/kg (calculated as the amount of paclitaxel) into the tail vein on the day after grouping (day 1) and on day 8.

The ratio of tumor volume on day 15 to tumor volume upon grouping (day 0) (tumor volume on day 15/tumor volume on day 0) was regarded as the relative tumor volume (RTV). Tumor growth inhibition (%) was determined using the mean relative tumor volume of each drug-administered group and the mean relative tumor volume of the tumor-bearing control group (not treated with drugs) according to the following formula 3.

Formula 3

(1-(relative tumor volume of drug-administered group)/(relative tumor volume of tumor-bearing control group)) × 100 (%)

The effect due to the combined use was analyzed using the relative tumor volume on day 15 according to the Intersection-Union Test (IUT) procedure.

The ratio of the difference in body weight between the mice on day 15 and the mice upon grouping (day 0) to the body weight of the mice on day 0 (body weight change; BWC, %) was determined as an index of systemic toxicity according to formula 4 below.

Formula 4

((Body weight of mice on day 15 – body weight of mice on day 0)/ body weight of mice on day 0) × 100(%)

Table 2 shows the obtained results.

Table 2

| Antitumor effect of TS-1/I-OHP/paclitaxel combination in human | | | | | | |
|---|---|---|---|---|---|---|
| gastric cancer SC-2 xenograft model | | | | | | |
| Drug | TS-1 (Tegafur Amount) Dose (mg/kg) | I-OHP Dose (mg/kg) | APX Preparation Dose (mg/kg) | Relative Tumor Volume (Mean) | Tumor Growth Inhibition (%) | Body Weight Change (%) |
| None | - | - | - | 10.26 | - | 3.19 |
| Tegafur + Gimeracil+ Oteracil Potassium+ Oxaliplatin (TS-1+I-OHP) | 5.0 | 5.0 | - | 5.39** | 47.5 | -1.71 |
| APX Preparation | - | - | 20.0 | 3.10** | 69.8 | 2.98 |
| APX Preparation | - | - | 40.0 | 2.37** | 76.9 | 7.15 |
| Tegafur + Gimeracil + Oteracil Potassium+ Oxaliplatin+ APX (TS-1+I-OHP+APX) | 5.0 | 5.0 | 20.0 | 2.26**## | 77.9 | 5.39 |
| Tegafur + Gimeracil + Oteracil Potassium+ Oxaliplatin+ APX (TS-1+I-OHP+APX) | 5.0 | 5.0 | 40.0 | 1.48**## | 85.6 | 5.36 |
| The symbol "**" indicates a significant difference from the control (not treated with drugs) (p<0.01). | | | | | | |

The symbol "##" indicates that analysis according to the IUT procedure revealed significant enhancing antitumor effect (p<0.01).

The results show that when the APX preparation was administered in combination with the TS-1/l-OHP combination therapy, a statistically significantly high antitumor effect was obtained, and toxicity (in particular, an increase in toxicity) that was evaluated by body weight loss was not observed.

Example 3

Tumor growth inhibition effect of the combined use of TS-1/l-OHP/paclitaxel on human gastric cancer cell line SC-2

Formulation of pharmaceutical preparations and administration of the preparations were performed in the same manner as in Example 2.

Including the day of grouping, the tumor diameter was measured two or three times a week for a total of 4 weeks including a two-week observation period after the completion of the drug administration, and a period of time until the mean relative tumor volume of each group reaches 4 (relative tumor volume 4 (day)) was calculated. The relative tumor volume 4 of the tumor-bearing control group was subtracted from the relative tumor volume 4 of each drug-administered group, and the obtained value was regarded as a growth delay period (day).

```
Formula 5

Relative tumor volume 4 of drug-administered group - relative tumor volume 4 of tumor-bearing control group (day)
```

Table 3 shows the obtained results.

Table 3

| Tumor growth inhibition effect of the combined use of TS-1/l-OHP/paclitaxel in human gastric cancer SC-2 xenograft model | | | | | |
|---|---|---|---|---|---|
| Drug | TS-1 (Tegafur Amount) Dose (mg/kg) | l-OHP Dose (mg/kg) | APX Preparation Dose (mg/kg) | Relative Tumor Volume 4 (Day) | Growth Delay Period (Day) |
| None | - | - | - | 8.2 | - |
| Tegafur + Gimeracil+ Oteracil Potassium+ Oxaliplatin (TS-1+l-OHP) | 5.0 | 5.0 | - | 10.3 | 2.2 |
| APX Preparation | - | - | 20.0 | 19.0 | 10.8 |
| APX Preparation | - | - | 40.0 | 22.6 | 14.4 |
| Tegafur + Gimeracil + Oteracil Potassium+ Oxaliplatin+ APX (TS-1+l-OHP+APX) | 5.0 | 5.0 | 20.0 | 25.2 | 17.0 |
| Tegafur + Gimeracil + Oteracil Potassium+ Oxaliplatin+ APX (TS-1+l-OHP+APX) | 5.0 | 5.0 | 40.0 | 27.6 | 19.4 |
| Relative tumor volume 4: time for tumor volume at the start of administration to quadruple Growth delay period: value obtained by subtracting the relative tumor volume 4 of the control group (not treated with drugs) from the relative tumor volume 4 of each drug-administered group. | | | | | |

The results show that the growth delay period of the TS-1/l-OHP combination therapy, a hitherto known technique, was 2.2 days and the growth delay periods of the APX preparation at 20 mg/kg and 40 mg/kg were 10.8 days and 14.4 days, respectively. The combined use of TS-1/l-OHP combination therapy and the APX preparation at 20 mg/kg or 40 mg/kg of the present invention showed growth delay periods of 17.0 days or 19.4 days, respectively, exhibiting a notably high growth delay effect that is unexpected from the hitherto known technique. These results suggest that the combined use of the TS-1/l-OHP combination therapy and paclitaxel results in excellent prolongation of life in the treatment of patients with tumors.

**Claims**

1. An antitumor agent comprising a combination of oxaliplatin, paclitaxel, and a combination drug containing tegafur, gimeracil, and oteracil potassium.

2. The antitumor agent according to claim 1, wherein the combination drug contains tegafur, gimeracil, and oteracil potassium at a molar ratio of tegafur:gimeracil:oteracil potassium = 1:0.4:1.

3. The antitumor agent according to any one of claims 1 and 2, which is in a pharmaceutical form comprising the combination drug containing tegafur, gimeracil, and oteracil potassium, a preparation containing oxaliplatin, and a preparation containing paclitaxel.

4. The antitumor agent according to claim 3, wherein the combination drug containing tegafur, gimeracil, and oteracil potassium is administered by oral administration, and the preparation containing oxaliplatin and the preparation containing paclitaxel are individually administered by an administration route selected from the group consisting of intravenously, intramuscularly, and subcutaneously.

5. The antitumor agent according to any one of claims 3 and 4, wherein the combination drug containing tegafur, gimeracil, and oteracil potassium, the preparation containing oxaliplatin, and the preparation containing paclitaxel are administered simultaneously or at one or more intervals.

6. The antitumor agent according to any one of claims 1 to 5, wherein oxaliplatin is contained at a daily dose of 80 to 150 mg/m$^2$, paclitaxel is contained at a daily dose of 50 to 500 mg/m$^2$, and the combination drug containing tegafur, gimeracil, and oteracil potassium is contained at a daily dose such that tegafur is contained in an amount of 60 to 120 mg/m$^2$.

7. The antitumor agent according to any one of claims 3 to 6, wherein the pharmaceutical form of paclitaxel is a nanoparticle preparation comprising paclitaxel encapsulated in albumin.

8. A kit comprising a combination drug containing tegafur, gimeracil, and oteracil potassium, a preparation containing oxaliplatin, and a preparation containing paclitaxel.

9. A method for treating a tumor, comprising administering oxaliplatin, paclitaxel, and a combination drug containing tegafur, gimeracil, and oteracil potassium, in combination.

10. Use of tegafur, gimeracil, oteracil potassium, oxaliplatin, and paclitaxel for tumour treatment that uses oxaliplatin, paclitaxel, and a combination drug containing tegafur, gimeracil, and oteracil potassium, in combination.

11. A combination of pharmaceutical compositions for tumor treatment, the combination comprising an antitumor composition containing tegafur, gimeracil, and oteracil potassium, an antitumor composition containing oxaliplatin, and an antitumor composition containing paclitaxel.

12. A combination drug containing tegafur, gimeracil, and oteracil potassium for use in tumor treatment in combination with oxaliplatin and paclitaxel.

13. A combination drug containing tegafur, gimeracil, and oteracil potassium for treating a patient with a tumor by administering the combination drug, oxaliplatin, and paclitaxel simultaneously or at one or more intervals.

14. A kit comprising a combination drug containing tegafur, gimeracil, and oteracil potassium, the kit further comprising a package insert, label, or instructions each stating that the combination drug, oxaliplatin, and paclitaxel are administered in combination for treating a patient with a tumor.

15. Paclitaxel for use in tumor treatment in combination with oxaliplatin and a combination drug containing tegafur, gimeracil, and oteracil potassium.

16. Paclitaxel for treating a patient with a tumor by administering paclitaxel, oxaliplatin, and a combination drug containing tegafur, gimeracil, and oteracil potassium simultaneously or at one or more intervals.

17. A kit comprising paclitaxel, the kit further comprising a package insert, label, or instructions each stating that paclitaxel, oxaliplatin, and a combination drug containing tegafur, gimeracil, and oteracil potassium are administered in combination for treating a patient with a tumor.

## INTERNATIONAL SEARCH REPORT

| International application No. |
|---|
| PCT/JP2013/057403 |

### A. CLASSIFICATION OF SUBJECT MATTER
*A61K31/53*(2006.01)i, *A61K31/282*(2006.01)i, *A61K31/337*(2006.01)i, *A61K31/4412*(2006.01)i, *A61K31/513*(2006.01)i, *A61P35/00*(2006.01)i, *A61P43/00*(2006.01)i

According to International Patent Classification (IPC) or to both national classification and IPC

### B. FIELDS SEARCHED

Minimum documentation searched (classification system followed by classification symbols)
A61K31/53, A61K31/282, A61K31/337, A61K31/4412, A61K31/513, A61P35/00, A61P43/00

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CA/REGISTRY/MEDLINE/EMBASE/BIOSIS(STN),
JSTPlus/JMEDPlus/JST7580(JDreamII)

### C. DOCUMENTS CONSIDERED TO BE RELEVANT

| Category* | Citation of document, with indication, where appropriate, of the relevant passages | Relevant to claim No. |
|---|---|---|
| X,Y | Yasuhiro ODERA et al., "Shokaki Gan Kagaku Ryoho no Key Drug", Gastroenterological Surgery Nursing, 2006, 11(9), pages 874 to 886 | 1-8,11-17 |
| X,Y | Hiroya TAKIUCHI, "Shinki Saibo Dokusei Koganzai no Kiso to Rinsho", G. I. Research, 2006, 14(1), pages 11 to 19 | 1-8,11-17 |
| X,Y | Hiroshi NEMOTO et al., "Shokaki (Shokudo · I · Daicho) Gan Kagaku Ryoho ni Taisuru Watashitachi no Torikumi", Iyaku no Mon, 2007, 47(3), pages 271 to 279 | 1-8,11-17 |
| X,Y | Narikazu BOKU, "DPD Inhibitory Fluoropyrimidine (DIF) in Gastrointestinal Malignancies", Biotherapy, 2003, 17(4), pages 361 to 367 | 1-8,11-17 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 08 April, 2013 (08.04.13) | 16 April, 2013 (16.04.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
| --- |
| PCT/JP2013/057403 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X<br>Y | Yasuhiro TERASAKI et al., "P3-089 Toin ni Okeru Genpatsu Fumei Gan 10 Rei no Kento", The 9th Annual Meeting of Japanese Society of Medical Oncology Program · Shorokushu, 2011, page 474 | 15,16<br>1-8,11-14,17 |
| P,A | SATOH, H. et al, S-1 for the treatment of gastrointestinal cancer, Expert Opinion on Pharmacotherapy, 2012.09, 13(13), pp.1943-1959 | 1-8,11-17 |

Form PCT/ISA/210 (continuation of second sheet) (July 2009)

## INTERNATIONAL SEARCH REPORT

| International application No. |
|---|
| PCT/JP2013/057403 |

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 9,10
   because they relate to subject matter not required to be searched by this Authority, namely:
   The claims 9,10 involve "methods for treatment of the human body or animal body by surgery or therapy".

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012059987 A
- WO 05120480 A

- JP 2614164 B
- JP 2003300878 A

**Non-patent literature cited in the description**

- *Cancer,* 2007, vol. 109, 33-40
- *J. Clin. Oncol.,* 2009, vol. 27 (15s), 4553

- *Oncology,* 2008, vol. 74 (1-2), 37-41